# EUROPÄISCHE PATENTANMELDUNG

(11) **EP 4 382 596 A1**
(43) Veröffentlichungstag der Anmeldung: **12.06.2024**
(21) Anmeldenummer: 24162649.8
(22) Anmeldetag: 18.10.2021
(51) Int. Cl.: C12M 1/18, C12Q 1/686, B01L 3/00, C12Q 1/70

(54) **KIT ZUR HERSTELLUNG EINER REAKTIONSLÖSUNG, VERFAHREN UND REAKTIONSLÖSUNG**

(30) Priorität: 20.10.2020 DE 202020004407 U
(62) Teilanmeldung aus: 21794540.1
(71) Anmelder: FRIZ Biochem GmbH, 82061 Neuried (DE)
(72) Erfinder: Hartwich, Gerhard, 82061 Neuried (DE); Wetzel, Lisa, 82061 Neuried (DE)
(74) Vertreter: Zeuner Summerer Stütz

(57) **Zusammenfassung**

Die Erfindung betrifft eine funktionalisierte Mikrotiterplatte für den direkten Nachweis von Pathogenen, vor allem Viren, und insbesondere von SARS-CoV-2 Viren, mittels reverser Transkription (RT) und/oder Polymerase-Kettenreaktion (PCR) einer Pathogen-codierenden Nukleinsäure direkt aus einem Untersuchungsmaterial ohne Präanalytik. Die Mikrotiterplatte enthält in einem Streifen oder einer Platte eine Mehrzahl von eine Öffnung aufweisenden Gefäßen. Eines oder mehrere der Gefäße sind funktionalisiert und weisen jeweils einen Vorlagebereich für Vorlagematerial und einen Aufnahmebereich für das Untersuchungsmaterial auf. Dabei ist vorgesehen dass,
- der Aufnahmebereich zur Aufnahme des Untersuchungsmaterials durch die Gefäßöffnung ausgelegt und eingerichtet ist, und
- der Vorlagebereich als Vorlagematerial
a) von einem Wachs umschlossene, funktionelle Reaktionschemikalien für den direkten Nachweis der Pathogen-codierenden Nukleinsäure,
b) eine Reaktionspufferlösung, und
c) eine Dichtungsschicht enthält, die die in dem Gefäß vorliegenden, von Wachs umschlossenen Reaktionschemikalien und die Reaktionspufferlösung bedeckt und zum Aufnahmebereich hin abdichtet.

## Beschreibung

Die Erfindung betrifft eine funktionalisierte Mikrotiterplatte für den direkten Nachweis von Pathogenen, vor allem Viren, und insbesondere von SARS-CoV-2 Viren, mittels reverser Transkription (RT) und/ oder Polymerase-Kettenreaktion (Polymerase Chain Reaction-PCR) einer Pathogen-codierenden Nukleinsäure direkt aus einem Untersuchungsmaterial ohne Präanalytik. Die Erfindung betrifft auch eine Reaktionslösung für den direkten Nachweis von Viren, insbesondere von SARS-CoV-2 Viren in einem Untersuchungsmaterial mittels RT-PCR ohne separate Präanalytik und ein Kit zur Herstellung einer solchen Reaktionslösung.

Die Coronavirus-Erkrankung (SARS-Coronaviren wie SARS-CoV-2, Erreger der Pandemie COVID-19) ist eine Infektionskrankheit, die von einem neu entdeckten Coronavirus ausgelöst wird. Das Virus wird vorwiegend durch Tröpfcheninfektion übertragen, wenn eine infizierte Person hustet, niest oder ausatmet. Die Symptome können von Fieber, Husten und Atembeschwerden bis hin zu Lungenentzündung und akutem Atemnotsyndrom reichen und bei komorbiden Personen zum Tod führen.

Die gegenwärtig zuverlässigste Testmethode beruht auf der quantitativen Reverse-Transkriptase-Polymerase-Kettenreaktion (oft als qRT-PCR, RTqPCR oder nur als PCR-Test bezeichnet), bei der Nukleinsäure aus SARS-CoV-2 RNA in Proben der oberen und unteren Atemwege von Personen, bei denen der Verdacht auf COVID-19 besteht, nachgewiesen wird. Die SARS-CoV-2 RNA ist im Allgemeinen in respiratorischen Proben während der akuten Phase der Infektion nachweisbar. Allerdings ist diese Nachweismethode insbesondere wegen der erforderlichen Präanalytik, also der Lyse der SARS-CoV-2 Viren und der Isolierung und Aufreinigung der codierenden Nukleinsäure, sehr komplex und zeitaufwendig.

Ausgehend davon liegt der Erfindung die Aufgabe zugrunde, die Nachteile des Stands der Technik zu vermeiden, und insbesondere Mittel und eine Vorrichtung anzugeben, die einen einfachen und schnellen Nachweis von Pathogenen, vor allem Viren, und insbesondere von SARS-CoV-2 Viren ermöglichen.

Diese Aufgabe wird durch die Merkmale der unabhängigen Ansprüche gelöst. Weiterbildungen der Erfindung sind Gegenstand der abhängigen Ansprüche.

In einem nicht beanspruchten Aspekt enthält die vorliegende Beschreibung eine funktionalisierte Mikrotiterplatte für den direkten Nachweis von Pathogenen, vor allem Viren, und insbesondere von SARS-CoV-2 Viren, mittels reverser Transkription (RT) und/ oder Polymerase-Kettenreaktion (PCR) einer Pathogen-codierenden Nukleinsäure direkt aus einem Untersuchungsmaterial ohne Präanalytik bereit, wobei die Mikrotiterplatte in einem Streifen oder einer Platte eine Mehrzahl von eine Öffnung aufweisenden Gefäßen enthält, und eines oder mehrere der Gefäße funktionalisiert sind und jeweils einen Vorlagebereich für Vorlagematerial und einen Aufnahmebereich für das Untersuchungsmaterial aufweisen. Dabei ist
- der Aufnahmebereich zur Aufnahme des Untersuchungsmaterials durch die Gefäßöffnung ausgelegt und eingerichtet, und
- enthält der Vorlagebereich als Vorlagematerial
   a) von einem Wachs umschlossene, funktionelle Reaktionschemikalien für den direkten Nachweis der Pathogen-codierenden Nukleinsäure,
   b) eine Reaktionspufferlösung, und
   c) eine Dichtungsschicht, die die in dem Gefäß vorliegenden, von Wachs umschlossenen Reaktionschemikalien und die Reaktionspufferlösung bedeckt und zum Aufnahmebereich hin abdichtet.

Die Dichtungsschicht ist vorzugsweise schmelzbar und insbesondere aus einem Wachs, einer Paraffinmischung oder Vaseline gebildet. In einer vorteilhaften Ausgestaltung ist die Dichtungsschicht elastisch, so dass sie bei mechanischer Belastung der Mikrotiterplatte nicht leicht bricht oder undicht wird.

Die funktionellen Reaktionschemikalien sind zweckmäßig getrocknet und durch die umschließende Wachsschicht vor Licht und Sauerstoff geschützt.

Unter der Aussage, dass die funktionalisierte Mikrotiterplatte dem direkten Nachweis von Pathogenen aus einem Untersuchungsmaterial ohne Präanalytik dient, ist insbesondere zu verstehen, dass die funktionalisierte Mikrotiterplatte zum Nachweis der Pathogene ohne separate Lyse der Pathogene und ohne Isolierung und Aufreinigung der Pathogen-codierenden Nukleinsäure eingerichtet ist.

In einer vorteilhaften Weiterbildung der funktionalisierten Mikrotiterplatte enthalten die funktionellen Reaktionschemikalien spezifische Primer und qPCR-Probes, dNTPs, MgCl₂, optional Agenzien zur Unterstützung der Lyse, optional Chelatbildner wie etwa ETDA (Ethylendiamintetraessigsäure), Polymerase, im Fall, dass als Pathogene RNA-Viren nachgewiesen werden sollen, zusätzlich Reverse Transkriptase, sowie Agenzien zur Reduktion von potentiellen Inhibitoren der Transkription und/ oder Polymerase-Reaktion.

Mit Vorteil enthalten die funktionellen Reaktionschemikalien ferner eine Interne-Kontroll-Substanz, bei RNA-Viren-Pathogenen insbesondere eine künstliche oder nicht natürlich vorkommende RNA-Sequenz, bei anderen Pathogenen insbesondere eine künstliche oder nicht natürlich vorkommende DNA-Sequenz.

Die Erfindung enthält einen Kit zur Herstellung einer Reaktionslösung für den direkten Nachweis von Viren, insbesondere von SARS-CoV-2 RNA in einem Untersuchungsmaterial mittels RT-PCR ohne separate Präanalytik, umfassend
- zu mischende Lösungen A und B, wobei
- Lösung A spezifische Primer und qPCR-Probes, dNTPs, MgCl₂, optional Agenzien zur Unterstützung der Lyse, optional Chelatbildner wie etwa ETDA (Ethylendiamintetraessigsäure), Polymerase und Reverse Transkriptase enthält, und
- Lösung B Agenzien zur Reduktion von potentiellen Inhibitoren der Transkription und/oder Polymerase-Reaktion, sowie eine Interne-Kontroll-Substanz, insbesondere eine künstliche oder nicht natürlich vorkommende RNA-Sequenz, enthält.

Die Erfindung enthält auch ein Verfahren zur Herstellung einer Reaktionslösung für den direkten Nachweis von Viren, insbesondere von SARS-CoV-2 Viren, in einem Untersuchungsmaterial mittels RT-PCR ohne separate Präanalytik, wobei ein Kit der oben beschriebenen Art bereitgestellt wird und die Lösungen A und B gemischt werden, um die genannte Reaktionslösung für den direkten Nachweis von Viren zu erhalten.

Die Erfindung enthält weiter eine Reaktionslösung für den direkten Nachweis von Viren, insbesondere von SARS-CoV-2 Viren, in einem Untersuchungsmaterial mittels RT-PCR ohne separate Präanalytik, erhalten durch eine Mischung der Lösungen A und B des Kits von Anspruch 1.

Die so erhaltene Reaktionslösung umfasst zwei Lösungsbestandteile A und B, wobei
- Lösungsbestandteil A spezifische Primer und qPCR-Probes, dNTPs, MgCl₂, optional Agenzien zur Unterstützung der Lyse, optional Chelatbildner wie etwa ETDA (Ethylendiamintetraessigsäure), Polymerase und Reverse Transkriptase enthält, und
- Lösungsbestandteil B Agenzien zur Reduktion von potentiellen Inhibitoren der Transkription und/ oder Polymerase-Reaktion, sowie eine Interne-Kontroll-Substanz, insbesondere eine künstliche oder nicht natürlich vorkommende RNA-Sequenz, enthält.

Die in den Lösungsbestandteilen A und B der Reaktionslösung bzw. den Lösungen A und B des Kits enthalten Chemikalien entsprechen dabei gerade den vorzugsweise im Vorlagebereich der Gefäße der funktionalisierten Mikrotiterplatte vorgelegten funktionellen Reaktionschemikalien.

Vorteile der Erfindung sowie ein Ausführungsbeispiel werden nachfolgend anhand der Figuren erläutert, bei deren Darstellung auf eine maßstabs- und proportionsgetreue Wiedergabe verzichtet wurde, um die Anschaulichkeit zu erhöhen.

Es zeigen:
- Fig. 1: schematisch eine erfindungsgemäße funktionalisierte Mikrotiterplatte mit einer Mehrzahl von Gefäßen, und

- Fig. 2: schematisch ein Gefäß der Mikrotiterplatte der Fig. 1 im Querschnitt im Detail.

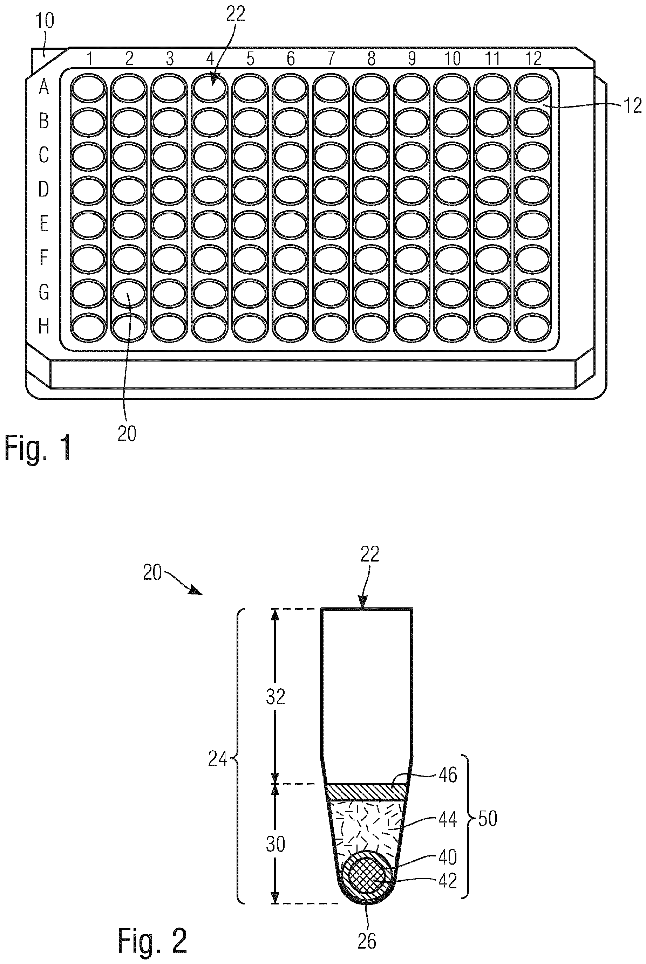

Als Hintergrund der Erfindung wird zunächst eine funktionalisierte Mikrotiterplatte für den Nachweis von SARS-CoV-2 Viren erläutert. Figur 1 zeigt hierzu eine Mikrotiterplatte 10 mit einer Platte 12, die eine Mehrzahl (beispielsweise 96) gleichartiger Gefäße 20 enthält, die jeweils am oberen Rand mit der Platte 12 verbunden sind.

Wie am besten in der Querschnittsdarstellung der Fig. 2 zu erkennen, haben die Gefäße 20 in der Ebene der Platte 12 eine von oben zugängliche Öffnung 22 und einen sich nach unten erstreckenden Körper 24, der unten durch einen Boden 26 verschlossen ist.

Als Besonderheit sind einige oder sogar alle Gefäße 20 der Mikrotiterplatte funktionalisiert, das heißt, sie weisen neben einem Aufnahmebereich 32 zur Aufnahme des Untersuchungsmaterials auch einen Vorlagebereich 30 mit einem spezifisch auf den Nachweis des gesuchten Pathogens, hier des SARS-CoV-2 Virus, abgestimmten Vorlagematerial 50 auf. Das Vorlagematerial 50 enthält dabei
a) von einem Wachs 40 umschlossene, funktionelle Reaktionschemikalien 42 für den direkten Nachweis der SARS-CoV-2-codierenden Nukleinsäure,
b) eine Reaktionspufferlösung 44, und
c) eine Dichtungsschicht 46, beispielsweise aus Wachs, einer Paraffinmischung, oder Vaseline, die die in dem Gefäß 20 vorliegenden, von

Wachs 40 umschlossenen Reaktionschemikalien 42 und die Reaktionspufferlösung 44 bedeckt und zum Aufnahmebereich 32 hin abdichtet.

Die funktionellen Reaktionschemikalien 42 liegen getrocknet vor und sind durch die umschließende Wachsschicht 40 vor Licht und Sauerstoff geschützt.

Für den gewünschten Nachweis von SARS-CoV-2 Viren enthalten die funktionellen Reaktionschemikalien spezifische Primer und qPCR-Probes, dNTPs, MgCl₂, optional Agenzien zur Unterstützung der Lyse, optional Chelatbildner wie etwa ETDA (Ethylendiamintetraessigsäure), Polymerase und Reverse Transkriptase, Agenzien zur Reduktion von potentiellen Inhibitoren der Transkription und/ oder Polymerase-Reaktion, sowie eine Interne-Kontroll-Substanz in Form einer eine künstlichen oder nicht natürlich vorkommende RNA-Sequenz.

Um das Funktionsprinzip des COVID-19 RT-PCR Tests näher zu erläutern, enthalten die funktionellen Reaktionschemikalien Nachweissonden, die spezifisch für SARS-CoV-2 sind und Sonden für die Detektion der Untergattung Sarbecovirus sowie eine interne Kontrolle. Die Sonden sind jeweils mit fluoreszierenden Reporter-Farbstoffen markiert, beispielsweise FAM für SARS-CoV-2, N-Gen; CalFluor Red 610 für Sarbecovirus, E-Gen; HEX für die interne Kontrolle. Jede Sonde verfügt zudem über einen zweiten Farbstoff, der als Quencher dient und die Fluoreszenzsignale der intakten Sonden unterdrückt. Während der PCR-Amplifikation hybridisieren die Sonden an eine spezifische Target-Sequenz, falls diese in der Probe vorhanden ist. Dies führt zur Spaltung der Sonde durch die 5'-zu-3'-Exonukleaseaktivität der DNA-Polymerase und damit zur Trennung der Reporter- und Quencher-Farbstoffe, was wiederum zu einem Anstieg des Fluoreszenzsignals führt. Mit jedem PCR-Zyklus werden zunehmend mehr gespaltene Sonden erzeugt und das Signal nimmt weiter zu. Jeder Reporter-Farbstoff wird bei einer definierten Wellenlänge gemessen, was den gleichzeitigen Nachweis und die Unterscheidung verschiedener Target-Sequenzen im Coronavirus sowie der internen Kontrolle erlaubt. Die Analyse erfolgt semi-quantitativ, indem Ct (Cycle threshold)-Werte verglichen werden. Der Ct-Wert beschreibt den Zyklus, in dem das Signal erstmals über einen bestimmten Schwellenwert ansteigt. Je mehr Target-Kopien (hier: SARS-CoV-2-Virus-RNA) in der Probe vorhanden waren, desto niedriger ist der Wert.

Um sicherzustellen, dass eine Patientenprobe keine RT-PCR-inhibierenden Substanzen enthält, ist jeder Reaktion eine Interne-Kontroll-Substanz zugefügt. Bei dieser wird ein künstliches RNA-Target, welches keine bekannten Homologien aufweist und im selben RT-PCR Ansatz enthalten ist, in cDNA umgeschrieben, amplifiziert und detektiert. So können falsch negative Testergebnisse durch Inhibition der RT-PCR ausgeschlossen werden.

Die Reaktionspufferlösung 44 enthält Additive zur Viruslyse, Verstärkung der PCR-Effizienz und Inhibition von RNasen. Dadurch können Patientenproben direkt und ohne vorherige RNA-Extraktion analysiert werden.

Als besonderen Vorteil bildet die funktionalisierte Mikrotiterplatte 10 ein gebrauchsfertiges System zum Nachweis von SARS-CoV-2 Viren ohne Präanalytik, da in dem Vorlagebereich 30 der Gefäße alle benötigten funktionellen Reaktionschemikalien 42 und auch der Reaktionspuffer 44 bereits geschützt vorliegen. Es müssen lediglich die unbehandelten Patientenproben als Untersuchungsmaterial in die Aufnahmebereiche 32 der funktionalisierten Gefäße 20 der Mikrotiterplatte 10 zugegeben werden. Die Analyse erfolgt dann direkt in einem qPCR Gerät, beispielsweise einem Roche LightCycler^{®} System (96/480 II) oder einem BioRad CFX96^{™} Dx qPCR Cycler.

Im qPCR Gerät schmelzen durch die für die RT bzw. PCR ohnehin erforderliche erhöhte Temperatur die Dichtungsschicht 46 und die Wachsschicht 40 im Vorlagebereich der Gefäße, so dass sich das Untersuchungsmaterial mit der Reaktionspufferlösung 44 und den funktionellen Reaktionschemikalien 42 vermischt und die gewünschte PCT bzw. RT-PCR durchgeführt werden kann.

Anstelle einer funktionalisierten Mikrotiterplatte mit den bereits vorgelegten funktionellen Reaktionschemikalien kann auch eine erfindungsgemäße Reaktionslösung verwendet werden, die dann allerdings von geschultem und autorisiertem Fachpersonal vor der Zugabe der Patientenprobe in die PCR-Reaktionsgefäße einer Mikrotiterplatte gefüllt werden muss.

Auch bei Verwendung einer solchen Reaktionslösung entfällt die Notwendigkeit einer separaten Präanalytik. Die Reaktionslösung enthält zwei Lösungsbestandteile A und B, wobei
- Lösungsbestandteil A spezifische Primer und qPCR-Probes, dNTPs, MgCl₂, optional Agenzien zur Unterstützung der Lyse, optional Chelatbildner wie etwa ETDA (Ethylendiamintetraessigsäure), Polymerase und Reverse Transkriptase enthält, und
- Lösungsbestandteil B Agenzien zur Reduktion von potentiellen Inhibitoren der Transkription und/ oder Polymerase-Reaktion, sowie eine Interne-Kontroll-Substanz, insbesondere eine künstliche oder nicht natürlich vorkommende RNA-Sequenz, enthält.

Wegen der kurzen Haltbarkeit der Reaktionslösung empfiehlt sich die jeweils bedarfsweise Herstellung der Reaktionslösung aus einem Kit mit zu mischenden Lösungen A und B, wobei
- Lösung A spezifische Primer und qPCR-Probes, dNTPs, MgCl₂, optional Agenzien zur Unterstützung der Lyse, optional Chelatbildner wie etwa ETDA (Ethylendiamintetraessigsäure), Polymerase und Reverse Transkriptase enthält, und
- Lösung B Agenzien zur Reduktion von potentiellen Inhibitoren der Transkription und/ oder Polymerase-Reaktion, sowie eine Interne-Kontroll-Substanz, insbesondere eine künstliche oder nicht natürlich vorkommende RNA-Sequenz, enthält.

### Illustrierende Ausgestaltungen:

Ausgestaltung 1: Funktionalisierte Mikrotiterplatte für den direkten Nachweis von Pathogenen, vor allem Viren, und insbesondere von SARS-CoV-2 Viren, mittels reverser Transkription (RT) und/ oder Polymerase-Kettenreaktion (PCR) einer Pathogen-codierenden Nukleinsäure direkt aus einem Untersuchungsmaterial ohne Präanalytik, wobei die Mikrotiterplatte in einem Streifen oder einer Platte eine Mehrzahl von eine Öffnung aufweisenden Gefäßen enthält, und eines oder mehrere der Gefäße funktionalisiert sind und jeweils einen Vorlagebereich für Vorlagematerial und einen Aufnahmebereich für das Untersuchungsmaterial aufweisen, wobei
- der Aufnahmebereich zur Aufnahme des Untersuchungsmaterials durch die Gefäßöffnung ausgelegt und eingerichtet ist, und
- der Vorlagebereich als Vorlagematerial
   a) von einem Wachs umschlossene, funktionelle Reaktionschemikalien für den direkten Nachweis der Pathogen-codierenden Nukleinsäure,
   b) eine Reaktionspufferlösung, und
   c) eine Dichtungsschicht enthält, die die in dem Gefäß vorliegenden, von Wachs umschlossenen Reaktionschemikalien und die Reaktionspufferlösung bedeckt und zum Aufnahmebereich hin abdichtet.

Ausgestaltung 2: Funktionalisierte Mikrotiterplatte nach Ausgestaltung 1, **dadurch gekennzeichnet, dass** die Dichtungsschicht schmelzbar ist und insbesondere aus einem Wachs, einer Paraffinmischung oder Vaseline gebildet ist.

Ausgestaltung 3. Funktionalisierte Mikrotiterplatte nach Ausgestaltung 1 oder 2, **dadurch gekennzeichnet, dass** die Dichtungsschicht elastisch ist.

Ausgestaltung 4. Funktionalisierte Mikrotiterplatte nach einer der Ausgestaltungen 1 bis 3, **dadurch gekennzeichnet, dass** die funktionellen Reaktionschemikalien getrocknet und durch die umschließende Wachsschicht vor Licht und Sauerstoff geschützt sind.

Ausgestaltung 5. Funktionalisierte Mikrotiterplatte nach einer der Ausgestaltungen 1 bis 4, **dadurch gekennzeichnet, dass** die Mikrotiterplatte zum Nachweis der Pathogenen ohne separate Lyse der Pathogene und ohne Isolierung und Aufreinigung der Pathogen-codierenden Nukleinsäure eingerichtet ist.

Ausgestaltung 6. Funktionalisierte Mikrotiterplatte nach einer der Ausgestaltungen 1 bis 5, **dadurch gekennzeichnet, dass** die funktionellen Reaktionschemikalien spezifische Primer und qPCR-Probes, dNTPs, MgCl₂, optional Agenzien zur Unterstützung der Lyse, optional Chelatbildner wie etwa ETDA (Ethylendiamintetraessigsäure), Polymerase, im Fall, dass als Pathogene RNA-Viren nachgewiesen werden sollen, zusätzlich Reverse Transkriptase, sowie Agenzien zur Reduktion von potentiellen Inhibitoren der Transkription und/ oder Polymerase-Reaktion enthalten.

Ausgestaltung 7. Funktionalisierte Mikrotiterplatte nach Ausgestaltung 6, **dadurch gekennzeichnet, dass** die funktionellen Reaktionschemikalien ferner eine Interne-Kontroll-Substanz enthalten, bei RNA-Viren-Pathogenen insbesondere eine künstliche oder nicht natürlich vorkommende RNA-Sequenz, bei anderen Pathogenen insbesondere eine künstliche oder nicht natürlich vorkommende DNA-Sequenz enthalten.

Ausgestaltung 8. Reaktionslösung für den direkten Nachweis von Viren, insbesondere von SARS-CoV-2 Viren, in einem Untersuchungsmaterial mittels RT-PCR ohne separate Präanalytik, umfassend zwei Lösungsbestandteile A und B, wobei
- Lösungsbestandteil A spezifische Primer und qPCR-Probes, dNTPs, MgCl₂, optional Agenzien zur Unterstützung der Lyse, optional Chelatbildner wie etwa ETDA (Ethylendiamintetraessigsäure), Polymerase und Reverse Transkriptase enthält, und
- Lösungsbestandteil B Agenzien zur Reduktion von potentiellen Inhibitoren der Transkription und/ oder Polymerase-Reaktion, sowie eine Interne-Kontroll-Substanz, insbesondere eine künstliche oder nicht natürlich vorkommende RNA-Sequenz, enthält.

Ausgestaltung 9. Kit zur Herstellung einer Reaktionslösung für den direkten Nachweis von Viren, insbesondere von SARS-CoV-2 RNA in einem Untersuchungsmaterial mittels RT-PCR ohne separate Präanalytik, umfassend
- zu mischende Lösungen A und B, wobei
- Lösung A spezifische Primer und qPCR-Probes, dNTPs, MgCl₂, optional Agenzien zur Unterstützung der Lyse, optional Chelatbildner wie etwa ETDA (Ethylendiamintetraessigsäure), Polymerase und Reverse Transkriptase enthält, und
- Lösung B Agenzien zur Reduktion von potentiellen Inhibitoren der Transkription und/ oder Polymerase-Reaktion, sowie eine Interne-Kontroll-Substanz, insbesondere eine künstliche oder nicht natürlich vorkommende RNA-Sequenz, enthält.

## Patentansprüche

1. Kit zur Herstellung einer Reaktionslösung für den direkten Nachweis von Viren, insbesondere von SARS-CoV-2 RNA in einem Untersuchungsmaterial mittels RT-PCR ohne separate Präanalytik, umfassend
- zu mischende Lösungen A und B, wobei
- Lösung A spezifische Primer und qPCR-Probes, dNTPs, MgCl₂, optional Agenzien zur Unterstützung der Lyse, optional Chelatbildner wie etwa ETDA (Ethylendiamintetraessigsäure), Polymerase und Reverse Transkriptase enthält, und
- Lösung B Agenzien zur Reduktion von potentiellen Inhibitoren der Transkription und/ oder Polymerase-Reaktion, sowie eine Interne-Kontroll-Substanz, insbesondere eine künstliche oder nicht natürlich vorkommende RNA-Sequenz, enthält.

2. Verfahren zur Herstellung einer Reaktionslösung für den direkten Nachweis von Viren, insbesondere von SARS-CoV-2 Viren, in einem Untersuchungsmaterial mittels RT-PCR ohne separate Präanalytik, wobei ein Kit nach Anspruch 1 bereitgestellt wird und die Lösungen A und B gemischt werden, um die genannte Reaktionslösung für den direkten Nachweis von Viren zu erhalten.

3. Reaktionslösung für den direkten Nachweis von Viren, insbesondere von SARS-CoV-2 Viren, in einem Untersuchungsmaterial mittels RT-PCR ohne separate Präanalytik, erhalten durch eine Mischung der Lösungen A und B des Kits von Anspruch 1.
